# EUROPÄISCHE PATENTSCHRIFT

(11) **EP 2 085 258 B1**
(45) Veröffentlichungstag und Bekanntmachung des Hinweises auf die Patenterteilung: **12.10.2011**
(21) Anmeldenummer: 08020513.1
(22) Anmeldetag: 26.11.2008
(51) Int. Cl.: B60H 3/00

(54) **Vorrichtung zur Beduftung eines Innenraumes**
Devise for dispersing fragrance into the interior of vehicles
Dispositif de diffusion de fragrance dans l'habitacle de véhicules

(30) Priorität: 31.01.2008 DE 102008007075
(43) Veröffentlichungstag der Anmeldung: 05.08.2009
(73) Patentinhaber: Behr GmbH & Co. KG, 70469 Stuttgart (DE)
(72) Erfinder: Fritsche, Uwe, 71686 Remseck am Neckar (DE); Kroner, Peter, 66482 Zweibrücken (DE); Lochmahr, Karl, 71665 Vaihingen/Enz (DE); Rais, Thomas, 71672 Marbach/Neckar (DE); Weber, Gerhard, 70469 Stuttgart (DE); Wolf, Walter, 71570 Oppenweiler-Zell (DE)

(56) Entgegenhaltungen:
- EP-A- 0 800 832
- WO-A-03/041750
- WO-A-2004/105814
- DE-A1- 10 327 122
- DE-A1-102005 025 755

## Beschreibung

Die Erfindung betrifft eine Vorrichtung zur Beduftung eines Innenraumes, insbesondere eines Fahrzeuginnenraumes, mit wenigstens einem Aufnahmebehälter zur Bevorratung eines Duftstoffes, der zum Austrag des Duftstoffes zumindest eine Öffnung aufweist.

Derartige Vorrichtungen sind aus dem Stand der Technik bekannt. Sie werden dazu eingesetzt, die Raumluft eines zum Aufenthalt von Menschen vorgesehen Raumes durch die Anreicherung mit Duftstoffen zu verbessern. Zur Steigerung des Wohlgefühls der sich in dem Raum aufhaltenden Personen kann die Raumluft beispielsweise mit beruhigenden oder stimulierenden Duftstoffen angereichert werden. Hierzu werden Vorrichtungen benötigt, die einen oder mehrere Duftstoffe bevorraten, um sie über einen längeren Zeitraum dosiert an die Raumluft abzugeben. Derartige Vorrichtungen werden auch in Fahrzeugen eingesetzt, zum Beispiel in die Lüftungskanäle von Fahrzeugklimaanlagen und/oder -belüftungsanlagen.

Da bei jedem Menschen der Geruchssinn unterschiedlich stark ausgeprägt ist, bedarf es zur Herstellung eines Wohlgefühls bei einer Vielzahl von Menschen der Möglichkeit, die Duftintensität individuell einzustellen bzw. zu variieren. Darüber hinaus tritt in der Regel ein Gewöhnungseffekt beim Menschen ein, der dazu führt, dass ein bestimmter Duft nach längerer Zeit kaum noch oder überhaupt nicht mehr wahrgenommen wird. Um diesem Effekt entgegenzuwirken, sollte die Beduftung beispielsweise in zeitlichen Intervallen erfolgen und/oder die Duftintensität in gewissen Zeitabständen verändert werden.

Aus der DE 103 27 122 A1 ist eine Beduftungsvorrichtung mit einer Dosiereinrichtung bekannt, die einen, einen Duftstoff aufnehmenden Hohlkörper mit mindestens einer Öffnung und ein Element mit mindestens einer weiteren Öffnung umfasst. Der Hohlköper und/oder das Element sind mittels eines Motors derart gegeneinander bewegbar bzw. ausrichtbar, dass die jeweiligen Öffnungen entweder einen Durchlass für den Duftstoff bilden oder der Durchlass für den Duftstoff versperrt ist. Um den Durchlass sicher zu viersperren, wird in der Druckschrift ferner vorgeschlagen, zwischen dem Hohlkörper und dem Element eine zusätzliche Abdichtung vorzusehen, die aus einem Gummiring, einem Vlies oder dergleichen bestehen kann.

Zur Befüllung der vorstehend beschriebenen Vorrichtung mit einem Duftstoff, wird in den Hohlkörper eine Duftstoffpatrone oder Duftstoffkartusche eingesetzt. Eine Änderung der Duftnote ist bei dieser Vorrichtung daher lediglich im Wege des Austausches der jeweilig eingesetzten Patrone oder Kartusche möglich.

DE 10 2005 025 755 offenbart eine Beduftungsvorrichtung nach dem Oberbegriff des Anspruchs 1.

Aufgabe der vorliegenden Erfindung ist es, eine alternative oder verbesserte Beduftungsvorrichtung bereitzustellen, die mindestens eine vorzugsweise jedoch mehrere Duftnoten dosiert abzugeben vermag und dabei eine kompakte Einheit bildet, die universell einsetzbar ist.

Die Aufgabe wird gelöst durch eine Vorrichtung mit den Merkmalen nach Anspruch 1. Bevorzugte Ausführungsformen werden in den Unteransprüchen beschrieben.

Erfindungsgemäß ist vorgesehen, dass zur Steuerung der Duftintensität und/oder der Duftmischung die Öffnung oder die Öffnungen über wenigstens ein am Aufnahmebehälter ausgebildetes klapp- und/oder verschwenkbares Deckelteil verschließbar ist oder sind. Das klapp- und/oder verschwenkbare Deckelteil kann dabei nicht nur in eine Schließstellung, sondern auch in verschiedene Öffnungsstellungen überführt werden. Je nach Öffnungsstellung des Deckelteils variiert auch der jeweils zur Verfügung stehende freie Öffnungsquerschnitt, so dass mehr oder weniger Duftstoff aus dem Aufnahmebehälter ausgetragen wird. Dabei kann der Austrag durch einen an der Öffnung bzw. den Öffnungen vorbei geführten Luftstrom oder durch Aufheizen eines den Duftstoff bindenden Mediums bewirkt werden.

Das Deckelteil ist an den Aufnahmebehälter angespritzt und/oder über ein Filmscharnier mit dem Aufnahmebehälter verbunden. Bevorzugt bestehen sowohl der Aufnahmebehälter als auch das Deckelteil aus Kunststoff. Um eine Klapp- und/oder Schwenkbewegung vollziehen zu können muss das Deckelteil angelenkt sein. Vorzugsweise ist das Gelenk als Filmschamier ausgebildet, da sich dieses besonders einfach in einem Arbeitsgang mit dem Deckelteil herstellen lässt. Alternativ kann das Gelenk aber auch in einem nachfolgenden Arbeitsgang ausgebildet werden, beispielsweise durch Materialdehnung, Materialabtrag oder durch Einschnitte.

Weiterhin bevorzugt wirkt zumindest ein Teilbereich des Deckelteiles mit einem Teilbereich des Aufnahmebehälters dichtend zusammen. Beispielsweise kann wenigstens eine Anlagefläche am Deckelteil und/oder am Aufnahmebehälter konisch und/oder derart profiliert ausgebildet sein, dass die Anlageflächen dichtend aneinander liegen und/oder nach Art einer Labyrinthdichtung dichtend ineinander greifen. Auf die Anordnung zusätzlicher Dichtungselemente zwischen Aufnahmebehälter und Deckelteil kann demnach verzichtet werden. Da die Dichtung in dem Deckelteil bzw. dem Aufnahmebehälter integriert ist, werden bei einem Austausch des Behälters auch die Dichtungen ausgetauscht. Dies ist insoweit von Vorteil, da Dichtungen im Laufe der Zeit durch Verschleiß ihre dichtende Wirkung verlieren. Mit Austausch des Behälters erfolgt somit auch gleichzeitig der Austausch der verschleißanfälligen Dichtungen.

Um das Deckelteil in eine Schließ- oder Öffnungsstellung zu überführen, ist es an einen Antrieb koppelbar oder umfasst einen solchen. Beispielsweise kann der Antrieb einer in einem Fahrzeug vorhandenen Klima- und/oder Belüftungsanlage als externer Antrieb genutzt werden. Bevorzugt weist die Vorrichtung jedoch einen eigenen Antrieb, zumindest jedoch eigene Antriebselemente auf, die mit einem externen Antrieb verbindbar sind.

Der exteme oder eigene Antrieb beinhaltet eine Steuerkulisse, in die ein an dem Deckelteil ausgebildeter Zapfen eingreift. Der Zapfen des Deckelteils wird in der Steuerkulisse zwangsgeführt. Beispielsweise kann die Steuerkulisse aus einer eine Steuerkurve aufweisenden Steuerscheibe bestehen, deren Bewegung eine Bewegung des Zapfens bewirkt, wobei das Deckelteil in eine Schließoder Öffnungsstellung überführt wird. Der Zapfen kann je nach Ausbildung und Anordnung der Steuerkulisse auf der Außenseite und/oder im Kantenbereich des Deckelteils angeordnet sein.

Nach einer alternativen Ausführungsform beinhaltet der Antrieb einen Exzenter oder einen Kurbeltrieb, wobei das Deckelteil über ein Betätigungselement mit dem Exzenter oder dem Kurbeltrieb koppelbar ist. Eine rotatorische Bewegung des Exzenters oder des Kurbeltriebs wird über das Betätigungselement derart auf das Deckelteil übertragen, dass das Deckelteil in eine Schließ- oder Öffnungsstellung überführt wird. Das Betätigungselement kann dabei ein- oder mehrteilig ausgebildet und mit dem Antriebselement und/oder mit dem Deckelteil fest verbunden sein. Das Betätigungselement kann beispielsweise einen Betätigungshebel und/oder einen Zapfen und/oder ein Rastelement, beispielsweise einen Rasthaken oder eine Rastnase umfassen.

Weiterhin bevorzugt beinhaltet der Antrieb zur Überführung des Deckelteils in eine Schließ- und/oder Öffnungsstellung wenigstens einen Mitnehmer als Betätigungselement. Der Mitnehmer ist Bestandteil des Antriebs und demnach nicht fest mit dem Deckelteil verbunden. Er steht jedoch zumindest zeitweise in Eingriff mit dem Deckelteil bzw. mit einem am Deckelteil angeordneten Betätigungselement, beispielsweise einem Bügel, einer Nut oder einer sonstigen eine Angriffsfläche bildenden Kante oder Ausnehmung. Der Mitnehmer kann beispielsweise als Zapfen, Rasthaken oder Rastnase ausgebildet sein. Die Form des Mitnehmers bedingt die Form der Aufnahme bzw. Angriffsfläche am Deckelteil und umgekehrt.

Vorteilhafterweise ist das Deckelteil mittels einer zusätzlichen Kraft, beispielsweise mittels Federkraft oder mittels Magnetkraft, in eine Schließ- oder Öffnungsstellung rückführbar. Die zusätzliche Kraft bewirkt somit eine Bewegung des Deckelteils, die der durch den Antrieb bewirkten Bewegung entgegengesetzt ist. Beispielsweise kann die Kraft einer Feder dazu eingesetzt werden, das Deckelteil in einer Schließstellung oder in einer Öffnungsstellung zu halten oder in eine solche zurückzuführen. Auf diese Weise kann die Antriebskraft auf das erforderliche Maß für die gegenläufige Bewegung reduziert werden.

Der Aufnahmebehälter einer erfindungsgemäßen Vorrichtung ist unabhängig von der jeweiligen konkreten Ausführungsform mit einem Duftstoff befüllbar, der wahlweise eine flüssige, viskose oder feste Form aufweisen kann. Beispielsweise kann der Duftstoff als Granulat vorliegen. Des Weiteren kann auch ein Vlies-, Watte- oder Schwammkörper als Duftstoffspeicher in den Aufnahmebehälter eingesetzt werden. Derartige Duftstoffspeicher vermögen in ihren Poren einen flüssigen, öligen oder wachsartigen Duftstoff über einen längeren Zeitraum zu bevorraten. Ist der Duftstoff verbraucht, wird der Duftstoffspeicher erneut mit Duftstoff benetzt oder befüllt. Bevorzugt wird in den Aufnahmebehälter jedoch wenigstens eine einen flüssigen, viskosen oder festen Duftstoff enthaltende Duftstoffpatrone oder Duftstoffkartusche eingesetzt, die nach Verbrauch des Duftstoffes gegen eine neue oder frisch beladene Patrone bzw. Kartusche einfach ausgetauscht wird.

Weiterhin bevorzugt weist die Vorrichtung mehrere Aufnahmebehälter auf, von denen jeder einen anderen Duftstoff bevorratet. Dabei bilden mehrere Aufnahmebehälter eine kompakte Einheit, die in einen Cupholder oder in ein Düsenmodul einer Fahrzeugklimaanlage und/oder -belüftungsanlage oder in ein Cockpit-, Dach- oder Türmodul eines Fahrzeugs einsetzbar ist. Kompakte Einheiten mit bis zu 12 Aufnahmebehältern sind beispielsweise auch in Form eines Trommelmagazins realisierbar.

Vorteilhafterweise ist die aus mehreren Aufnahmebehältern bestehende kompakte Einheit an einen gemeinsamen Antrieb koppelbar, so dass zur Auswahl der Duftnote ein Deckelteil eines bestimmten Aufnahmebehälters oder zur Steuerung der Duftmischung zeitgleich mehrere Deckelteile mehrerer Aufnahmebehälter in eine Schließ- oder Öffnungsstellung überführbar sind. Auf diese Weise lassen sich auch individuelle Duftnoten komponieren.

Der Antrieb umfasst hierzu die vorstehend bereits beschriebenen Antriebsund/oder Steuerungselemente wie Steuerkulisse, Exzenter oder Kurbeltrieb sowie die hiermit zusammenwirkenden Betätigungselemente und/oder Mitnehmer.

Weiterhin bevorzugt ist jeder Aufnahmebehälter oder jede in einen Aufnahmebehälter eingesetzte Duftstoffpatrone zur Steuerung der Duftmischung codiert. Die Codierung kann beispielsweise über einen an dem Aufnahmebehälter angebrachten Barcode erfolgen.

Nach einer bevorzugten Ausführungsform umfasst die kompakte Einheit einen eigenen Antrieb und/oder eigene Antriebs- und Steuerungselemente. Weiterhin bevorzugt umfasst die Einheit einen Schrittmotor. Darüber hinaus kann die Einheit einen Lüfter und/oder eine Ionisationseinheit zur Luftreinigung umfassen. Anstelle eines Schrittmotors als Antrieb kann ein Antriebselement, wie beispielsweise eine Steuerscheibe, auch über einen Freilauf an den Lüfter gekoppelt sein. Weitere mögliche Antriebsarten können ein Relais, Elemente aus Formgedächtnislegierungen, Spindeln, Steuerkeile, Hydraulik- oder Pneumatikzylinder oder ein Getriebe als Antriebselemente umfassen.

Da die Duftstoffe irgendwann verbraucht sind, ist es erforderlich, dass jeder Aufnahmebehälter neu befüllt oder ausgetauscht werden kann. Vorzugsweise sind hierzu die Aufnahmebehälter einer kompakten Einheit lösbar miteinander und/oder einer gemeinsamen Haltevorrichtung verbunden. Die Haltevorrichtung kann beispielsweise aus einer gemeinsamen Grundplatte, einem Gehäuse oder einer Gitterstruktur bestehen, in die die Aufnahmebehälter einfach eingesteckt oder eingeschoben werden. Auf diese Weise lassen sich Module herstellen, die an den zur Verfügung stehenden Bauraum anpassbar und somit universell einsetzbar sind. Zudem ist jeder Aufnahmebehälter einzeln aus dem Modul zur Neubefüllung oder zum Austausch entnehmbar. Mit dem Aufnahmebehälter werden auch Deckelteil und Dichtung ausgetauscht, so dass verschleißanfällige Teile keiner separaten Wartung bedürfen.

Bevorzugte Ausführungsformen der Erfindung werden anhand der Zeichnungen nachfolgen näher beschrieben. Es zeigen:
- Fig. 1: eine perspektivische Darstellung eines ersten Ausführungsbei- spiels,
- Fig. 2: einen Ausschnitt aus Fig. 1,
- Fig. 3: eine Draufsicht auf die Vorrichtung nach Fig. 1,
- Fig. 4: eine Draufsicht auf ein weiteres Ausführungsbeispiel,
- Fig. 5: eine perspektivische Darstellung der Vorrichtung nach Fig. 4,
- Fig. 6: eine perspektivische Darstellung eines Antriebselementes,
- Fig. 7: eine perspektivische Darstellung einer Vorrichtung mit Aufnahme- behälter und Antrieb,
- Fig. 8: eine perspektivische Darstellung der Vorrichtung nach Fig. 7 mit einem Gehäusemantel,
- Fig. 9: eine perspektivische Darstellung der Vorrichtung nach den Fig. 7 und 8 mit einem zusätzlichen Deckelteil,
- Fig. 10: eine perspektivische Darstellung eines weiteren Ausführungsbei- spiels,
- Fig. 11: eine perspektivische Darstellung der Vorrichtung nach Fig. 10 aus einer anderen Perspektive,
- Fig. 12: eine perspektivische Darstellung der Vorrichtung nach Fig. 10 oh- ne Gehäuse,
- Fig. 13: eine perspektivische Darstellung der Vorrichtung nach Fig. 11 oh- ne Gehäuse,
- Fig. 14: eine perspektivische Darstellung der Vorrichtung nach Fig. 13 oh- ne Lüfter,
- Fig. 15: eine perspektivische Darstellung der Vorrichtung nach Fig. 14 oh- ne Steuerscheibe,
- Fig. 16: eine Ansicht der Vorrichtung nach Fig. 14,
- Fig. 17: eine perspektivische Darstellung der Vorrichtung nach Fig. 14,
- Fig. 18a-b: jeweils eine Ansicht der Vorrichtung nach Fig. 14,
- Fig. 19a-b: jeweils eine Ansicht einer weiteren Ausführungsform,
- Fig. 20: eine schematische Schnittansicht einer weiteren Ausführungs- form,
- Fig. 21: eine schematische Schnittansicht einer weiteren Ausführungs- form,
- Fig. 22a-d: jeweils einen schematischen Detailschnitt durch eine Ausfüh- rungsform einer Dichtung,
- Fig. 23a-b: jeweils eine perspektivische Skizze einer weiteren Ausführungs- form eines Betätigungselementes,
- Fig. 24: eine perspektivische Skizze einer weiteren Ausführungsform eines Betätigungselementes,
- Fig. 25a-b: jeweils eine schematische Schnittansicht einer weiteren Ausfüh- rungsform eines Antriebselementes,
- Fig. 26a-b: jeweils eine schematische Schnittansicht einer weiteren Ausfüh- rungsform eines Betätigungselementes,
- Fig. 27: eine schematische Skizze einer weiteren Ausführungsform,
- Fig. 28a-b: jeweils eine schematische Skizze einer weiteren Ausführungs- form.

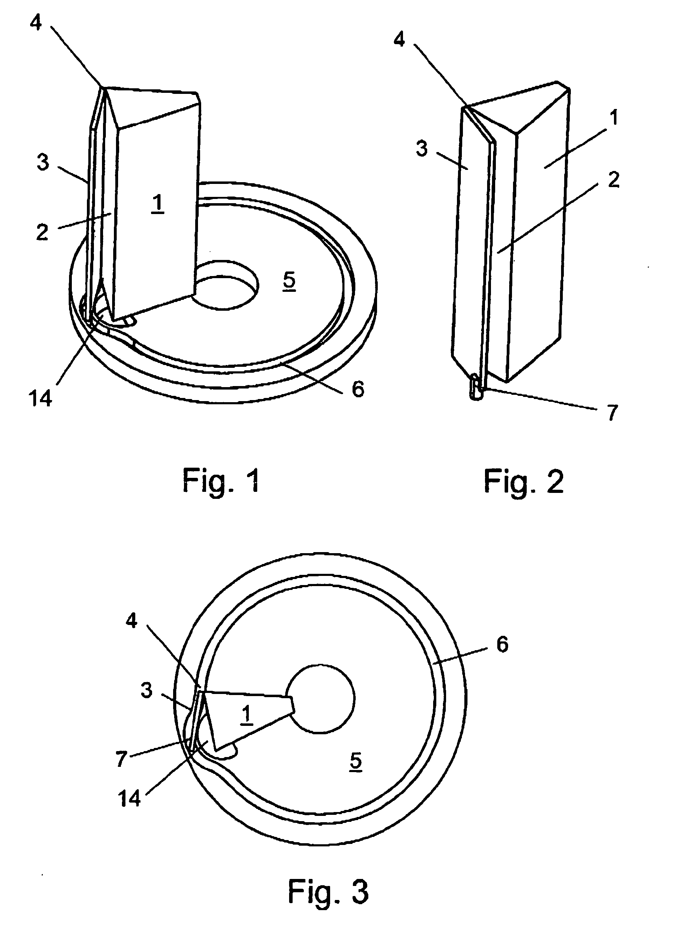

Ein erstes Ausführungsbeispiel der erfindungsgemäßen Vorrichtung ist in den Fig. 1 bis 3 dargestellt. Fig. 2 zeigt einen Aufnahmebehälter 1 mit einer trapezförmigen Grundrissform, der sich über eine senkrecht zur Grundfläche angeordnete Längsseite erstreckende Öffnung 2 aufweist. An dem Aufnahmebehälter 1 ist ein Deckelteil 3 ausgebildet, das sich über ein Filmscharnier 4 derart verschwenken lässt, dass es eine Schließ- sowie verschiedene Öffnungsstellungen mit jeweils einem unterschiedlich großen freien Öffnungsquerschnitt einnehmen kann. Der Aufnahmebehälter 1 dient der Bevorratung eines Duftstoffes, der beispielsweise in Form einer Duftstoffkartusche in den Aufnahmebehälter 1 einsetzbar ist. Zum Einsetzen wird das Deckelteil 3 in eine Öffnungsposition bewegt, so dass sich die Kartusche einlegen lässt.

Das Deckelteil 3 weist im unteren Kantenbereich einen Zapfen 7 auf, der bei Einsetzen eines Aufnahmebehälters 1 in ein mehrere Aufnahmebehälter 1 umfassendes Duftspendermodul in Eingriff mit einer Steuerkurve 6 einer Steuerscheibe 5 gebracht wird. Beispielsweise erlaubt das vorliegende Ausführungsbeispiel die Anordnung von bis zu 12 gleichen Aufnahmebehältern. Jeder Aufnahmebehälter 1 weist dabei ein Deckelteil 3 mit einem Zapfen 7 auf, der in Eingriff mit der Steuerkurve 6 zu bringen ist. Die Steuerkurve 6 der Steuerscheibe 5 ist derart ausgelegt, dass eine Drehbewegung der Steuerscheibe 5 gegenüber den Aufnahmebehältern 1 jeweils einen Zapfen 7 eines Deckelteils 3 in einen sich radial erweiternden Steuerkurvenabschnitt führt, so dass das jeweilige mit diesem Zapfen verbundene Deckelteil 3 zunächst in eine Öffnungs- und dann wieder in eine Schließposition bewegt wird. Jeweils ein Deckelteil 3 eines Aufnahmebehälters 1 lässt sich auf diese Weise öffnen und wieder schließen. Im Bereich des sich radial aufweitenden Steuerkurvenabschnitts weist die Steuerscheibe eine Öffnung 14 auf, durch die zum Austrag des Duftstoffes ein Luftstrom an der Öffnung 2 des Aufnahmebehälters 1 vorbeigeführt werden kann.

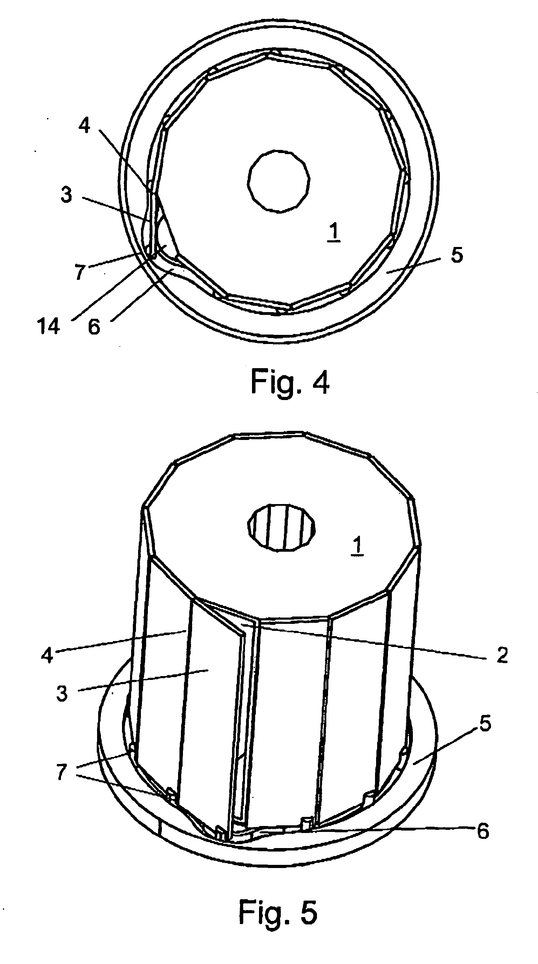

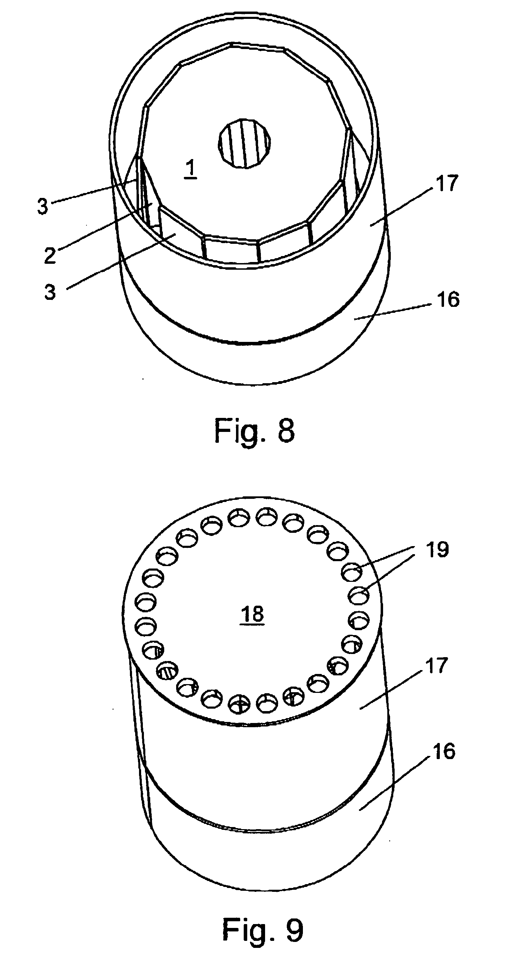

Den Fig. 4 bis 9 ist eine weitere Ausführungsform einer erfindungsgemäßen Vorrichtung zu entnehmen. Im Unterschied zu der vorstehend beschriebenen Ausführungsform weist der Aufnahmebehälter 1 eine polygonförmige Grundrissfläche mit 12 Seitenkanten auf, wobei sich über jede senkrecht zu einer Seitenkante angeordnete Längsseite eine Öffnung 2 erstreckt, die mittels eines Deckelteils 3 verschließbar ist. Jedes Deckelteil 3 weist wiederum einen Zapfen 7 auf, der in Eingriff mit einer Steuerkurve 6 einer Steuerscheibe 5 steht. Entsprechend dem Ausführungsbeispiel der Fig. 1 bis 3 bewirkt eine Drehung der Steuerscheibe 5 gegenüber dem Aufnahmebehälter 1, dass ein Deckelteil 3 geöffnet und wieder geschlossen wird. Eine im sich radial aufweitenden Steuerkurvenabschnitt angeordnete Öffnung 14 ermöglicht wiederum, dass ein Luftstrom zwischen geöffnetem Deckelteil 3 und Aufnahmebehälter 1 vorbeigeführt werden kann. Indem durch jede Öffnung 2 des Aufnahmebehälters 1 eine einen anderen Duftstoff enthaltende Duftstoffpatrone oder -kartusche eingesetzt werden kann, können bis zu 12 verschiedene Duftnoten zur Auswahl gestellt werden. Durch Drehung der Steuerscheibe 5 wird der sich radial aufweitende Steuerkurvenabschnitt und die Öffnung 14 zu dem jeweiligen Deckelteil 3 bewegt, hinter der sich die den jeweiligen Duftstoff enthaltende Patrone oder Kartusche befindet.

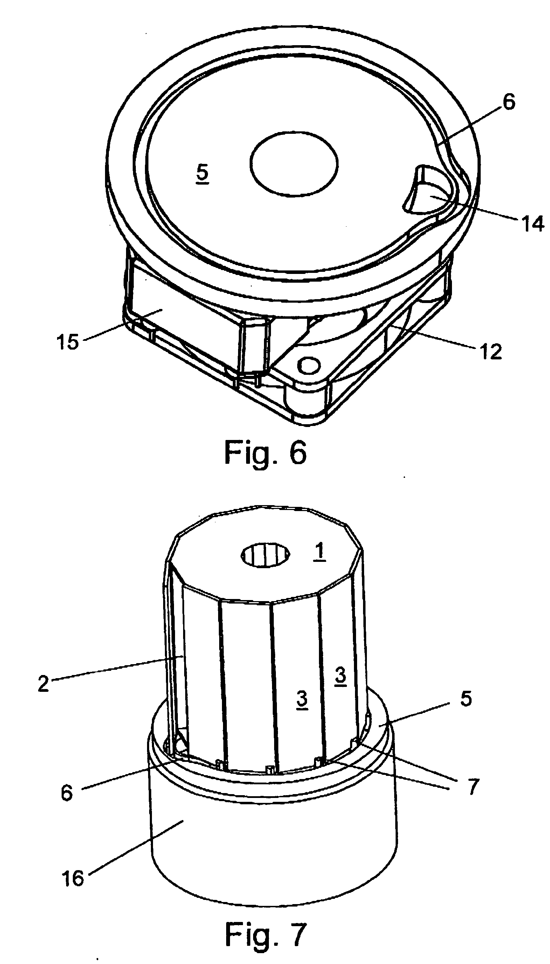

Um eine Drehbewegung der Steuerscheibe 5 zu bewirken, weist die Vorrichtung einen Schrittmotor 15 als zusätzliches Antriebselement auf, der unterhalb der Steuerscheibe 5 angeordnet ist (Fig. 6). Unter dem Schrittmotor 15 ist vorliegend zudem ein Lüfter 12 zur Erzeugung eines Luftstromes angeordnet, der über die Öffnung 14 der Steuerscheibe 5 an einer Öffnung 2 des Aufnahmebehälters 1 zum Austrag eines Duftstoffes vorbeigeführt wird.

Der Schrittmotor 15 und der Lüfter 12 können, wie in Fig. 7 dargestellt, in einem eigenen Gehäuseteil 16 angeordnet sein und somit eine untere Baueinheit bilden, die mit dem Aufnahmebehälter 1 als obere Baueinheit fest verbunden ist. Zwischen oberer und unterer Baueinheit ist die Steuerscheibe 5 drehbeweglich gelagert. Auch die obere, den Aufnahmebehälter 1 umfassende Baueinheit kann ein weiteres Gehäuseteil 17 umfassen, das oberseitig von einem Öffnungen 19 aufweisenden Deckelteil 18 abgedeckt wird. Die Öffnungen 19 leiten den mit Duftstoffen angereicherten Luftstrom direkt oder indirekt in den zu beduftenden Innenraum.

Bei dem Ausführungsbeispiel nach den Fig. 4 bis 9 ermöglicht die Anordnung sich radial erstreckender Trennwände (nicht dargestellt) innerhalb des Aufnahmebehälters 1 die Ausbildung von einzelnen Kammern, die jeweils einem bestimmten Deckelteil 3 zugeordnet sind. Dabei können die Deckelteile 3 über ein Filmscharnier 4 an den jeweiligen Trennwänden angelenkt sein.

Aufgrund der im Wesentlichen zylindrischen Form lässt sich die vorstehend beschriebene Vorrichtung in einfacher Weise in einen Lüftungskanal einer Fahrzeugkilmaanlage und/oder einer -belüftungsanlage einsetzen. Die Luftströmung kann dabei anlagenseitig erzeugt werden, so dass ein zusätzlicher Lüfter 12 entfallen kann. Folglich lässt sich die Vorrichtung besonders bauraumschonend mit einer als Trommelmagazin ausgebildeten oberen Baueinheit ausführen.

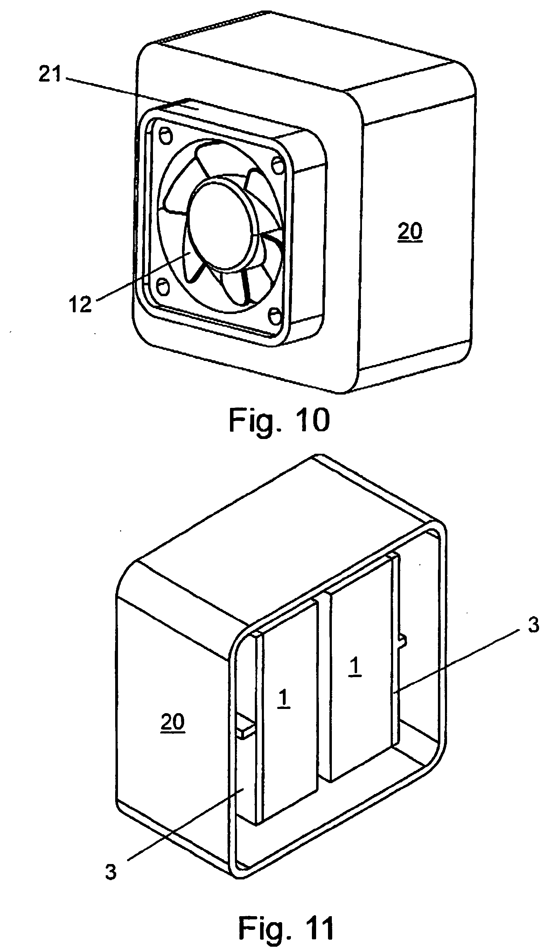

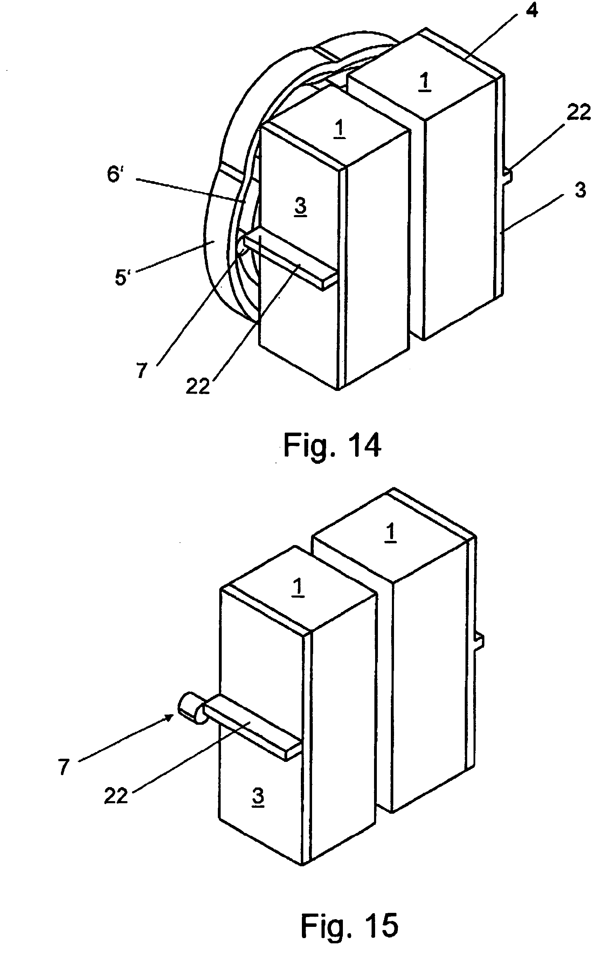

Die Fig. 10 bis 18 zeigen ein weiteres Ausführungsbeispiel einer erfindungsgemäßen Vorrichtung, das aufgrund seiner äußeren Abmessungen insbesondere für den Einbau in einen Bauraum geringer Tiefe, beispielsweise einer Türverkleidung geeignet ist.

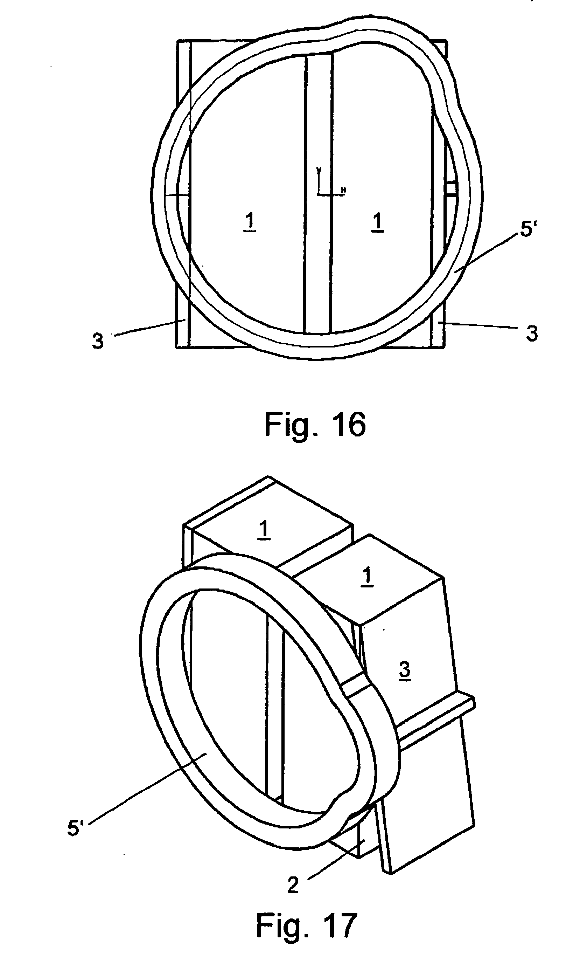

Bei diesem Ausführungsbeispiel sind zwei Aufnahmebehälter 1 aufrecht nebeneinander in einem Gehäuse 20 angeordnet, das rückseitig einen angesetzten Gehäuseabschnitt 21 zur Aufnahme eines Lüfters 12 aufweist. Die beiden Aufnahmebehälter 1 bilden jeweils eine im Wesentlichen quaderförmige Kammer aus, in die eine Duftstoffpatrone oder Duftstoffkartusche einsetzbar ist. Über jeweils eine Längsseite eines Aufnahmebehälters 1 erstreckt sich ein Deckelteil 3, das über ein Filmschamier 4 derart an dem Aufnahmebehälter 1 angelenkt ist, dass sich das Deckelteil 3 aufklappen lässt und somit eine Öffnung 2 freigibt (Fig. 17). Die Ausbildung der Aufnahmekörper und deren Anordnung zueinander sind nur beispielhaft dargestellt und können variieren. Beispielsweise kann das Deckelteil auch oben, unten, vorne oder hinten statt -wie dargestellt- seitlich angeordnet sein.

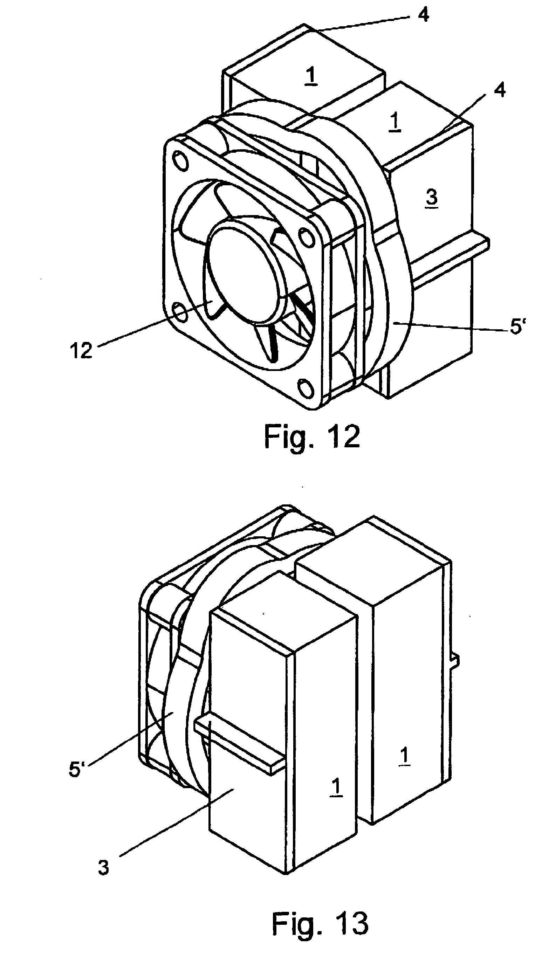

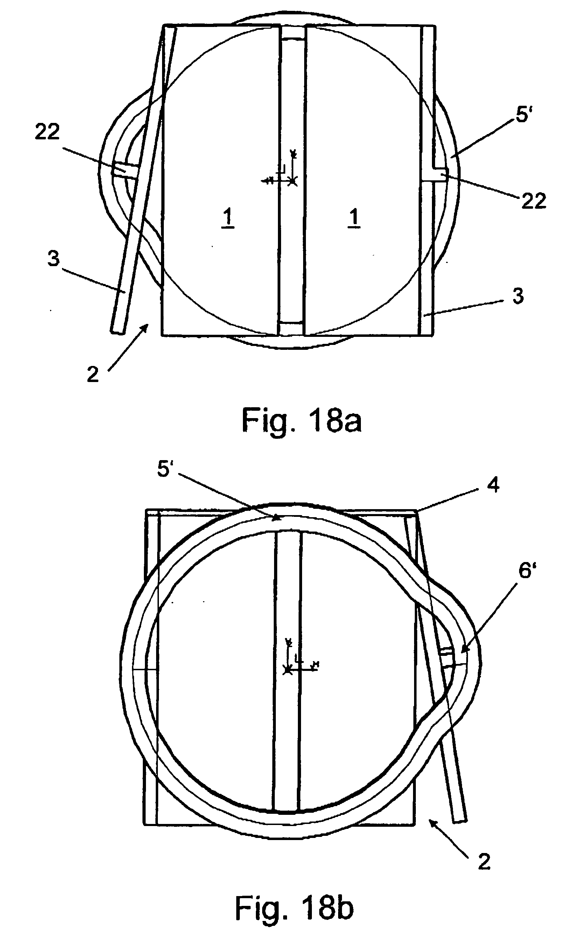

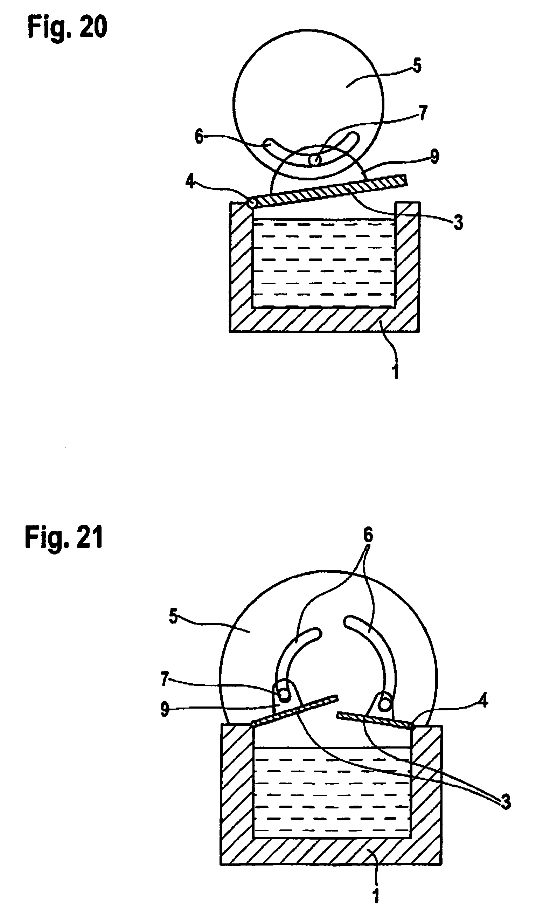

Den Darstellungen ohne Gehäuse 20 der Fig. 12 und 13 ist zu entnehmen, dass zwischen Lüfter 12 und den beiden Aufnahmebehältern 1 eine ringförmige Steuerscheibe 5' als Antriebselement angeordnet ist. Der Ringkörper 5' beschreibt die Form einer Steuerkurve 6' mit einem sich radial aufweitenden Ringabschnitt. Anstelle eines Ringkörpers 5' könnte auch eine Steuerscheibe 5 vorgesehen sein, die beispielsweise auch mehr als nur eine Steuerkurve 6 aufweisen kann. Dabei ist nicht erforderlich, dass die Steuerkurven umlaufend ausgebildet sind (entsprechend den Beispielen der Fig. 20 und 21). An einem sich quer zur Längsrichtung über dem Deckelteil 3 erstreckenden Bügel 22 befindet sich zum Ringkörper 5' hin ein Zapfen 7, der in Eingriff mit der Steuerkurve 6' des Ringkörpers 5' gebracht werden kann. Wird nunmehr der Ringkörper 5' in eine Drehbewegung versetzt, so dass der Zapfen 7 entlang der Steuerkurve 6' geführt wird, bewirkt der sich radial aufweitende Ringabschnitt, dass der Zapfen 7 derart bewegt wird, dass er das Deckelteil 3 zunächst in eine Öffnungsstellung und dann zurück in eine Schließstellung überführt. Wahlweise kann somit einer der beiden Aufnahmebehälter geöffnet und damit der darin enthaltene Duftstoff ausgetragen werden (Fig. 18a und b).

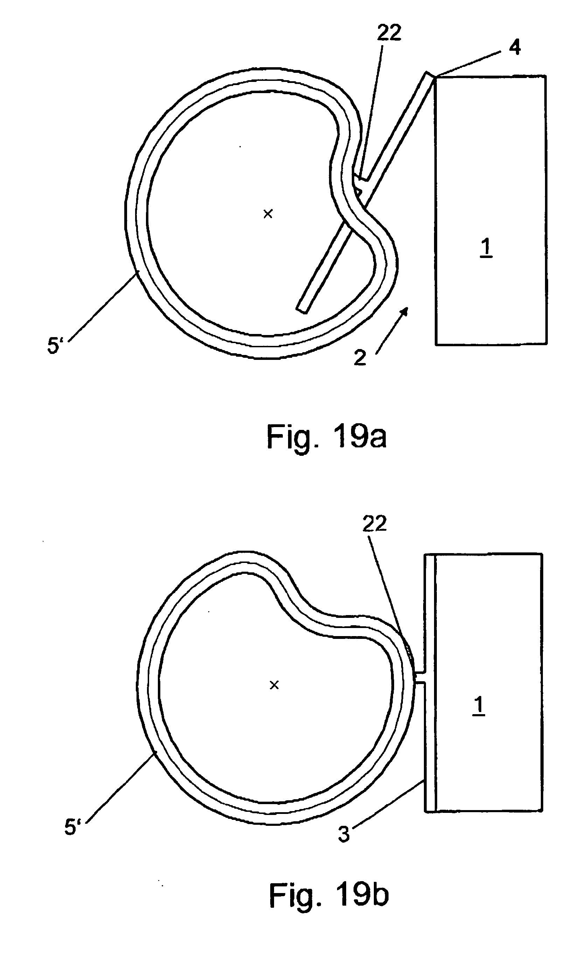

Um eine noch flacher bauende Ausführungsform zu erhalten, kann, wie in den Fig. 19a und b angedeutet, die ringförmige Steuerscheibe 5' auch zwischen zwei Aufnahmebehältern 1 angeordnet sein. In diesem Fall liegen sich die beiden Deckelteile 3 jeweils der Steuerscheibe 5' zugewandt- gegenüber.

Den Fig. 20 bis 28 sind weitere Ausführungsbeispiele der Erfindung oder Details solcher zu entnehmen.

Bei den Ausführungsformen nach den Fig. 20 und 21 weist der Aufnahmebehälter entweder ein ein- oder zweiteiliges Deckelteil 3 auf. Jeder Teil eines Deckelteils 3 ist über ein Filmscharnier 4 mit dem jeweiligen Aufnahmebehälter 1 verbunden. An jedem Teil ist zudem außenseitig ein Betätigungselement 9 in Form eines Bügels mit einem Zapfen 7 angeordnet, der in eine Steuerkurve 6 einer Steuerscheibe 5 eingreift. Bei einer Drehbewegung der Steuerscheibe 5 wird der Zapfen 7 entlang der Steuerkurve 6 geführt, wobei das Deckelteil 3 in eine Öffnungs- oder Schließstellung verschwenkt wird. Die Steuerscheibe 5 vollzieht dabei keine vollständige Drehung, da die Steuerkurven 6 bei den beiden Beispielen nicht umlaufend ausgebildet sind. Zusätzlich ist in einer nicht dargestellten Variante die Anordnung einer Trennwand etwa mittig im Behälter 1 nach Fig. 21 denkbar. Der Behälter wäre gemäß dieser Variante mit zwei unterschiedlichen Duftstoffen befüllbar, und die Größe der zugehörigen Öffnungen mit dem zweiteiligen Deckelteil 3 getrennt und/oder gemeinsam steuerbar.

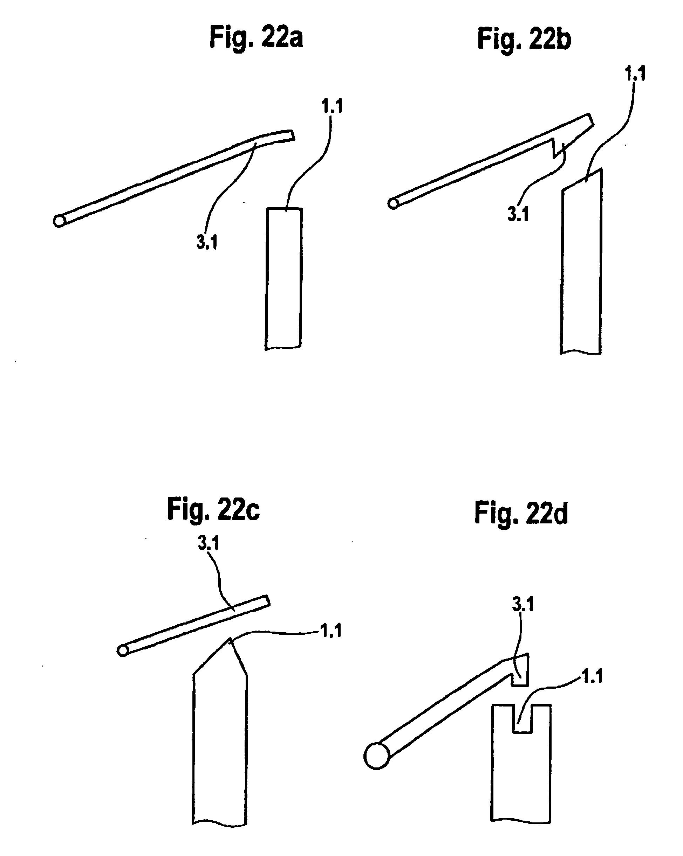

Die Fig. 22a bis 22d zeigen Beispiele eines anderen Details der Erfindung. Bevorzugt sind Deckelteil 3 und Aufnahmebehälter 1 derart ausgebildet, dass ein Teilbereich 3.1 des Deckelteils 3 mit einem Teilbereich 1.1 des Aufnahmebehälters 1 dichtend zusammenwirkt. Nach Fig. 22a können beide Teilbereiche 3.1 und 1.1 flach ausgebildet sein und in der Schließstellung des Deckelteils 3 dicht aneinander liegen. Eine dichtende Wirkung weisen auch die Beispiele der Fig. 22b und 22c auf, bei denen der Teilbereich 3.1 und/oder der Teilbereich 1.1 konisch verlaufend ausgebildet sind. Die Teilbereiche 3.1 und 1.1 können aber auch als Labyrinthdichtung entsprechend der Fig. 22d oder ähnlich ausgebildet sein.

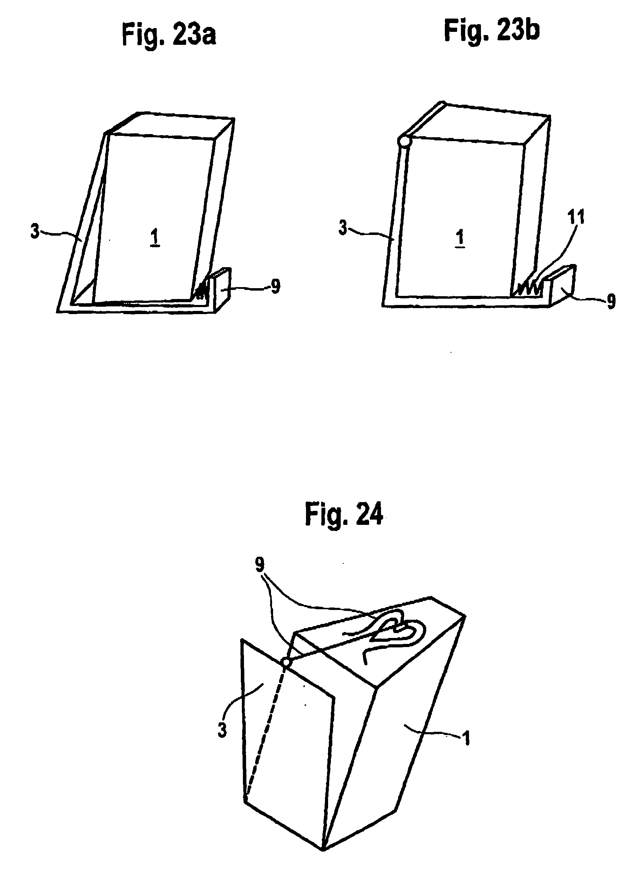

Den Fig. 23 bis 26 sind Beispiele verschiedener Betätigungselemente 9 zu entnehmen, die sowohl an einem Deckelteil 3, als auch an einem Antriebselement angeordnet sein können.

Bei den Fig. 23a und 23b weist das Deckelteil 3 einen Betätigungshebel 9 auf, der den Aufnahmebehälter 1 derart umgreift, dass sein freies Ende auf der dem Deckelteil 3 gegenüberliegenden Seite des Aufnahmebehälters in einem Abstand zu diesem zum Liegen kommt. Das freie Ende des Betätigungshebels 9 wird durch die Federkraft einer Feder 11 auf Abstand zum Aufnahmebehälter 1 gehalten. Um das Deckelteil 3 in eine Öffnungsstellung zu verschwenken, bedarf es der Überwindung der Federkraft der Feder 11.

Bei dem in Fig. 24 dargestellten Beispiel ist an dem Aufnahmebehälter 1 ein erstes Rastelement in Form einer Herzkurve und an dem Deckelteil 3 ein zweites Rastelement mit einem Haken angeordnet, der in der Herzkurve des ersten Rastelementes geführt ist. Herzkurve und Haken wirken nach Art eines Push-Push-Systems zusammen, wodurch sich das Deckelteil 3 öffnen und schließen lässt.

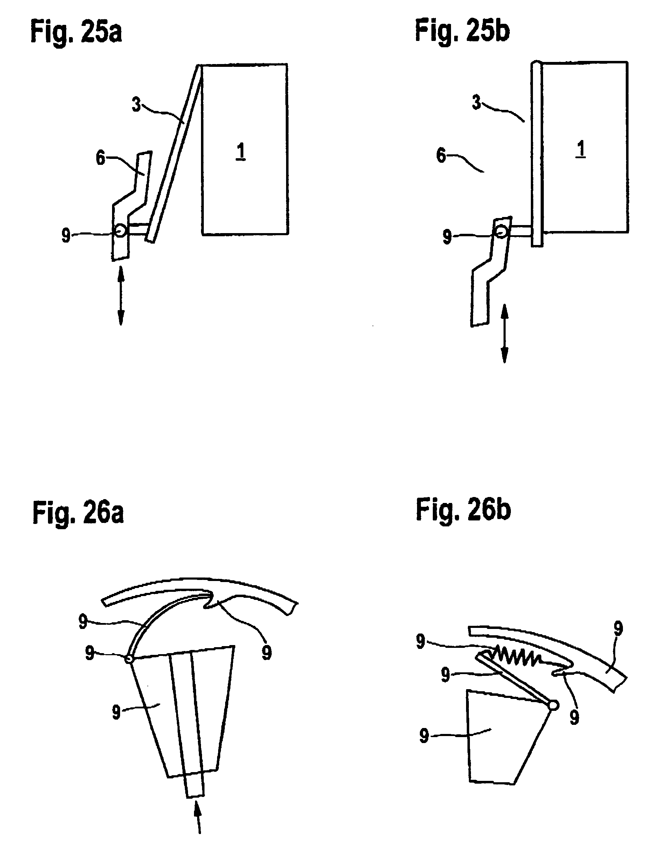

Die Fig. 25a und 25b zeigen ein Ausführungsbeispiel mit einer Steuerkulisse als Antriebselement. Als Betätigungselement 9 ist ein Zapfen am Deckelteil 3 vorgesehen, der in einer Steuerkurve 6 geführt ist Auf diese Weise kann das Deckelteil in eine Öffnungsstellung (Fig. 25a) und eine Schließstellung (Fig. 25b) bewegt werden.

Die Ausführungsbeispiele der Fig. 26a und 26b weisen jeweils einen mit einem Antrieb in Verbindung stehenden Mitnehmer 10 auf, der bei einer Drehbewegung des Mitnehmers 10 um den Aufnahmebehälter 1 das Deckelteil 3 erfasst und in eine Öffnungs- oder Schließstellung mitnimmt. Eine zusätzliche Feder 11 kann eine dieser Bewegung entgegen wirkende Kraft ausüben und sicherstellen, dass das Deckelteil 3 in seine Ausgangsstellung zurückfindet (Fig. 26b). Es können auch verschiedene Betätigungselemente kombiniert werden. Beispielsweise kann eine Feder 11 in Verbindung mit einem Betätigungshebel 9 entsprechend der Fig. 23a und 23b das über den Mitnehmer 10 bewegte Deckelteil 3 in seine Ausgangsstellung zurückführen (Fig. 26a).

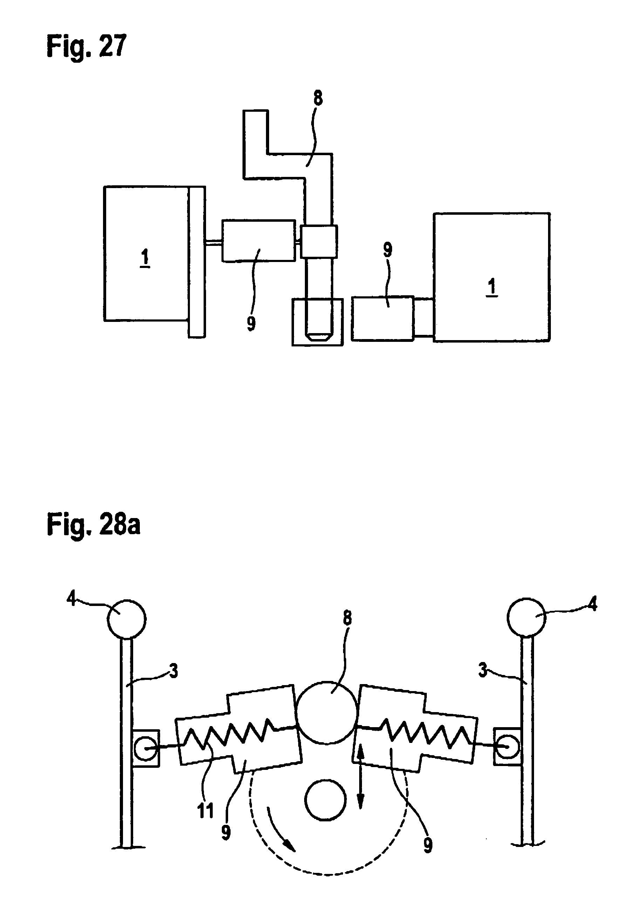

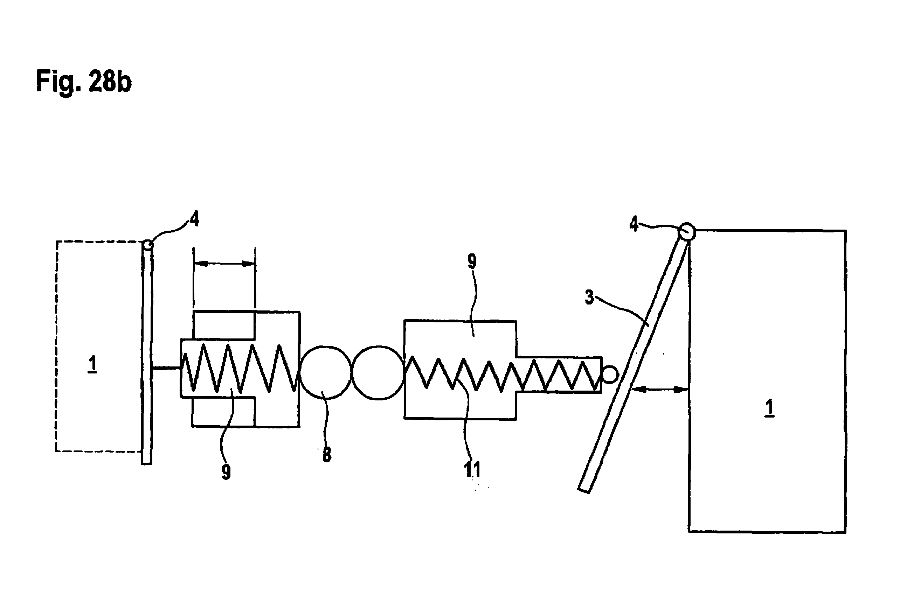

Eine weitere Kombination aus Antriebs- und Betätigungselement zeigt Fig. 27, bei der ein Exzenter 8 über Betätigungselemente 9 das Deckelteil 3 in eine Schließ- oder Öffnungsstellung überführt. Einen Exzenter 8 und Betätigungselemente 9 weist auch die Ausführungsform entsprechend der Fig. 28a und 28b auf. Zusätzlich umfassen die Betätigungselemente 9 jeweils eine Feder 11, mittels derer das Deckelteil 3 in die jeweilige Ausgangsstellung rückführbar ist.

Die dargestellten Ausführungsformen zeigen, dass es eine Vielzahl an Variationsmöglichkeiten gibt, eine am Aufnahmebehälter für den Austrag des Duftstoffes vorgesehene Öffnung über ein klapp- und/oder verschwenkbares Deckelteil im Sinne der vorliegenden Erfindung zu verschließen.

## Patentansprüche

1. Vorrichtung zur Beduftung eines Innenraumes, insbesondere eines Fahrzeuginnenraumes, mit wenigstens einem Aufnahmebehälter (1) zur Bevorratung eines Duftstoffes, der zum Austrag des Duftstoffes wenigstens eine Öffnung (2) aufweist, wobei zur Steuerung der Duftintensität und/oder der Duftmischung die Öffnung (2) oder die Öffnungen über wenigstens ein am Aufnahmebehälter (1) ausgebildetes, klapp- und/oder verschwenkbares Deckelteil (3) verschließbar ist oder sind, wobei das Deckelteil (3) an den Aufnahmebehälter (1) angespritzt und/oder über ein Filmschamier (4) mit dem Aufnahmebehälter verbunden ist, **dadurch gekennzeichnet, dass** das Deckelteil (3) zur Überführung in eine Schließ- oder Öffnungsstellung an einen Antrieb koppelbar ist oder einen Antrieb umfasst und der Antrieb eine Steuerkulisse, vorzugsweise eine Steuerkurve (6) aufweisende Steuerscheibe (5) beinhaltet, in die ein an dem Deckelteil (3) ausgebildeter Zapfen (7) eingreift.

2. Vorrichtung nach Anspruch 1, **dadurch gekennzeichnet, dass** zumindest ein Teilbereich (3.1) des Deckelteils (3) mit einem Teilbereich (1.1) des Aufnahmebehälters (1) dichtend zusammenwirkt.

3. Vorrichtung nach Anspruch 1 oder 2, **dadurch gekennzeichnet, dass** der Antrieb einen Exzenter (8) oder einen Kurbeltrieb beinhaltet, wobei das Deckelteil (3) über ein Betätigungselement (9) mit dem Exzenter oder dem Kurbeltrieb koppelbar sind.

4. Vorrichtung nach einem der vorhergehenden Ansprüche, **dadurch gekennzeichnet, dass** der Antrieb wenigstens einen Mitnehmer (10) zur Überführung des Deckelteils (3) in eine Schließ- und/oder Öffnungsstellung beinhaltet.

5. Vorrichtung nach einem der vorhergehenden Ansprüche, **dadurch gekennzeichnet, dass** das Deckelteil (3) mittels einer zusätzlichen Kraft, vorzugsweise mittels Federkraft einer Feder (11) oder mittels Magnetkraft, in eine Schließ- oder Öffnungsstellung rückführbar ist.

6. Vorrichtung nach einem der vorhergehenden Ansprüche, **dadurch gekennzeichnet, dass** in den Aufnahmebehälter (1) wenigstens eine Duftstoffpatrone oder Duftstoffkartusche einsetzbar ist.

7. Vorrichtung nach einem der vorhergehenden Ansprüche, **dadurch gekennzeichnet, dass** mehrere Aufnahmebehälter (1) eine kompakte Einheit bilden, die in einen Cupholder oder in ein Düsenmodul einer Fahrzeugklimaanlage und/oder -belüftungsanlage oder in ein Cockpit- oder Türmodul eines Fahrzeugs einsetzbar ist.

8. Vorrichtung nach Anspruch 7, **dadurch gekennzeichnet, dass** die aus mehreren Aufnahmebehältern (1) bestehende kompakte Einheit derart an einen gemeinsamen Antrieb koppelbar ist, dass zur Auswahl der Duftnote ein Deckelteil (3) eines bestimmten Aufnahmebehälters (1) oder zur Steuerung der Duftmischung zeitgleich mehrere Deckelteile (3) mehrerer Aufnahmebehälter (1) in eine Schließ- oder Öffnungsstellung überführbar sind.

9. Vorrichtung nach Anspruch 7 oder 8, **dadurch gekennzeichnet, dass** jeder Aufnahmebehälter (1) oder jede in einen Aufnahmebehälter eingesetzte Duftstoffpatrone zur Steuerung der Duftmischung codiert ist.

10. Vorrichtung nach einem der Ansprüche 7 bis 9, **dadurch gekennzeichnet, dass** die kompakte Einheit einen eigenen Antrieb und/oder eigene Antriebs- und Steuerungselemente umfasst.

11. Vorrichtung nach einem der Ansprüche 7 bis 10, **dadurch gekennzeichnet, dass** die kompakte Einheit einen Lüfter (12) und/oder eine Ionisationseinheit zur Luftreinigung umfasst.

12. Vorrichtung nach einem der Ansprüche 7 bis 11, **dadurch gekennzeichnet, dass** die Aufnahmebehälter (1) einer kompakten Einheit lösbar miteinander und/oder mit einer gemeinsamen Haltevorrichtung verbunden sind.

## Claims

1. A device for dispersing fragrance into an interior, in particular an interior of a vehicle, comprising at least one receptacle (1) for storing a fragrance, which includes at least one opening (2) for the discharge of the fragrance, wherein, in order to control the fragrance intensity and/or the fragrance mixture, the opening (2) or the openings can be closed using at least one cover part (3) which is formed on the receptacle (1) and can be folded and/or swiveled, wherein the cover part (3) is integrally extruded onto the receptacle (1) and/or is connected to the receptacle via a film hinge (4), **characterized in that** the cover part (3) can be coupled to a drive or comprises a drive for transfer to a closed position or an open position, and the drive contains a control gate, preferably a control disk (5) having a control curve (6), into which a pin (7) formed on the cover part (3) engages.

2. The device according to claim 1, **characterized in that** at least one subregion (3.1) of the cover part (3) interacts with a subregion (1.1) of the receptacle (1) in a sealing manner.

3. The device according to claim 1 or 2, **characterized in that** the drive contains an eccentric (8) or a crank mechanism, wherein the cover part (3) can be coupled to the eccentric or the crank mechanism via an actuating element (9).

4. The device according to one of the preceding claims, **characterized in that** the drive comprises at least one driving element (10) for transferring the cover part (3) into a closed position and/or open position.

5. The device according to one of the preceding claims, **characterized in that** the cover part, (3) can be returned to a closed position and/or open position using an additional force, preferably using spring force of a spring (11), or magnetic force.

6. The device according to one of the preceding claims, **characterized in that** at least one fragrance cartridge or fragrance cylinder can be inserted into the receptacle (1).

7. The device according to one of the preceding claims, **characterized in that** a plurality of receptacles (1) forms a compact unit which can be inserted into a cup holder or a nozzle module of a vehicle air conditioning system and/or ventilation system, or into a cockpit or door module of a vehicle.

8. The device according to claim 7, **characterized in that** the compact unit composed of a plurality of receptacles (1) can be coupled to a common drive in such a way that, to select the fragrance, a cover part (3) of a certain receptacle (1) can be transferred to a closed position or open position, or, to control the fragrance mixture, a plurality of cover parts (3) of a plurality of receptacles (1) can be transferred simultaneously to a closed position or open position.

9. The device according to claim 7 or 8, **characterized in that** every receptacle (1) or every fragrance cartridge inserted into a receptacle is coded to control the fragrance mixture.

10. The device according to one of the claims 7 to 9, **characterized in that** the compact unit comprises a separate drive and/or separate drive and control elements.

11. The device according to one of the claims 7 to 10, **characterized in that** the compact unit comprises a fan (12) and/or an ionization unit for cleaning the air.

12. The device according to one of the claims 7 to 11, **characterized in that** the receptacles (1) of a compact unit are detachably interconnected and/or are connected to a common holding device.

## Revendications

1. Dispositif servant à parfumer un habitacle, en particulier l'habitacle d'un véhicule, comprenant au moins un réservoir (1) servant au stockage d'un parfum, réservoir qui présente, pour la diffusion du parfum, au moins une ouverture (2), où l'ouverture (2) ou les ouvertures servant à réguler l'intensité du parfum, et / ou le mélange parfumé, peut ou peuvent être obturée (s) par au moins une partie (3) formant couvercle configurée sur le réservoir (1) en étant rabattable et / ou pivotante, où la partie (3) formant couvercle est moulée par injection sur le réservoir (1) et / ou assemblée avec le réservoir par une charnière pelliculaire (4),
**caractérisé en ce que** la partie (3) formant couvercle, pour passer dans une position de fermeture ou d'ouverture, peut être couplée à un mécanisme d'entraînement ou bien comprend un mécanisme d'entraînement, et le mécanisme d'entraînement comporte une coulisse de commande, de préférence un plateau porte-came (5) présentant une came de commande (6), plateau porte-came dans lequel s'engage un tenon (7) configuré sur la partie (3) formant couvercle.

2. Dispositif selon à revendication 1, **caractérisé en ce qu'**au moins une zone partielle (3.1) de la partie (3) formant couvercle coopère de façon étanche avec une zone partielle (1.1) du réservoir (1).

3. Dispositif selon la revendication 1 ou 2, **caractérisé en ce que** le mécanisme d'entraînement comporte un excentrique (8) ou un entraînement par manivelle, où la partie (3) formant couvercle (3) peut être couplée à l'excentrique ou à l'entraînement par manivelle, via un élément d'actionnement (9).

4. Dispositif selon l'une quelconque des revendications précédentes, **caractérisé en ce que** le mécanisme d'entraînement comprend au moins un élément d'entraînement (10) servant à faire passer la partie (3) formant couvercle, dans une position de fermeture et / ou d'ouverture.

5. Dispositif selon l'une quelconque des revendications précédentes, **caractérisé en ce que** la partie (3) formant couvercle peut être ramenée en position de fermeture ou d'ouverture, au moyen d'une force supplémentaire, de préférence par la force élastique d'un ressort (11) ou par une force magnétique.

6. Dispositif selon l'une quelconque des revendications précédentes, **caractérisé en ce qu'**au moins une recharge de parfum ou cartouche de parfum peut être introduite dans le réservoir (1).

7. Dispositif selon l'une quelconque des revendications précédentes, **caractérisé en ce que** plusieurs réservoirs (1) forment un ensemble compact qui peut être introduit dans un porte-gobelet ou dans un module de buse d'un système de climatisation et / ou d'un système de ventilation d'un véhicule, ou bien dans un module de poste de conduite ou de porte d'un véhicule.

8. Dispositif selon la revendication 7, **caractérisé en ce que** l'ensemble compact se composant de plusieurs réservoirs (1) peut être couplé à un mécanisme de d'entraînement commun de manière telle, que pour le choix de la note parfumée, une partie (3) formant couvercle d'un réservoir détermine (1) peut passer dans une position de fermeture ou d'ouverture, ou bien pour la commande du mélange parfumé, plusieurs parties (3) formant les couvercles de plusieurs réservoirs (1) peuvent passer en même temps dans une position de fermeture ou d'ouverture.

9. Dispositif selon la revendication 7 or 8, **caractérisé en ce que** chaque réservoir (1) ou chaque recharge due parfum introduite dans un réservoir est codé (e) pour à commande du mélange parfumé.

10. Dispositif selon l'une quelconque des revendications 7 à 9, **caractérisé en ce que** l'ensemble compact comprend un propre mécanisme d'entraînement et / ou des élément de commande et de mécanisme d'entraînement propres.

11. Dispositif selon l'une quelconque des revendications 7 à 10, **caractérisé en ce que** l'ensemble compact comprend une ventilateur (12) et / ou un ensemble d'ionisation pour la purification de l'air.

12. Dispositif selon l'une quelconque des revendications 7 à 11, **caractérisé en ce que** les réservoirs (1) d'un ensemble compact sont assemblés les uns aux autres de façon amovible et / ou avec un dispositif de fixation commun.
